(19) **Europäisches Patentamt**

**European Patent Office**

**Office européen des brevets**

(11) **EP 0 774 265 B1**

(12) **EUROPEAN PATENT SPECIFICATION**

(45) Date of publication and mention
of the grant of the patent:
**24.09.2003 Bulletin 2003/39**

(51) Int Cl.⁷: $A61L\ 17/08$, $A61L\ 17/14$

(21) Application number: **95118169.2**

(22) Date of filing: **17.11.1995**

(54) **Coated gut suture**

Beschichtetes Katgut Nahtmaterial

Fil de suture en boyau revêtu

(84) Designated Contracting States:
**DE ES FR GB IT**

(43) Date of publication of application:
**21.05.1997 Bulletin 1997/21**

(73) Proprietor: **United States Surgical Corporation**
**Norwalk, Connecticut 06856 (US)**

(72) Inventors:
• **Totakura, Nagabhushanam**
**North Haven, CT 06473 (US)**
• **Roby, Mark S.**
**Killingworth, CT 06419 (US)**
• **Jiang, Ying**
**North Haven, CT 06473 (US)**
• **Bennett, Steven L.**
**New Haven, CT 06513 (US)**

(74) Representative: **Marsh, Roy David et al**
**Hoffmann Eitle,**
**Patent- und Rechtsanwälte,**
**Arabellastrasse 4**
**81925 München (DE)**

(56) References cited:
**EP-A- 0 128 043**       **EP-A- 0 628 587**
**US-A- 3 896 814**       **US-A- 4 201 216**
**US-A- 5 037 429**

EP 0 774 265 B1

**Description**

<u>BACKGROUND</u>

1. <u>Technical Field</u>

The present disclosure relates to a coated gut suture and, more particularly, to a coated gut suture which is capable of being dry packaged.

2. <u>Background of the Related Art</u>

Absorbable sutures are manufactured from natural or synthetic materials. Some of the earliest absorbable sutures were made of collagenous material taken from sheep intestines. Such sutures are still in use today and are commonly referred to as "catgut" or simply "gut" sutures or ligatures. In the present specification, the term "catgut" or "gut" suture refers to a collagen based suture or ligature of any type or origin. Gut sutures may be prepared in the form of threads or strands that are undesirably stiff before subsequent treatment which renders them flexible or pliable.

A suture having a good degree of flexibility and pliability can conform closely to body tissue without undue pressure. Good flexibility and pliability enhance the degree to which a suture can be tied down, knotted and securely placed in a desired position.

Various attempts have been made to modify and optimize the physical characteristics of gut sutures. For example, tubing fluids, i.e., liquids which are used to condition gut sutures to achieve or enhance flexibility and pliability, have been used to preserve gut sutures. Tubing fluids typically contain an alcohol such as isopropyl alcohol and a relatively small percentage of water. Examples of tubing fluids are found in U.S. Patent Nos. 1,239,690, 2,394,054, 2,519,404, 2,524,772, and 2,694,487. Ideally, the tubing fluid aids the gut suture to retain its flexibility and pliability without adversely affecting the strength and overall integrity of the suture.

Commercially available gut sutures are immersed in tubing fluid, sterilized and supplied to surgeons in packages or tubes which contain tubing fluid. The alcohol and water present in the tubing fluid keep the suture flexible and pliable as long as they remain in contact with the suture. As the tubing fluid evaporates, the suture loses its flexibility and pliability which may affect handling characteristics.

In addition to tubing fluids, various suture coatings which adhere to the surface of the suture have been developed in an attempt to maintain flexibility and control swelling and fraying. Such coatings are also intended to improve the handling characteristics of sutures and maximize run-down performance. For example, U.S. Patent No. 3,942,532 discloses a suture coating composition obtained by polymerizing lactones such as ε-caprolactone in the presence of a polymethylenediol. U.S. Patent No. 4,624,256 discloses a suture coating composition containing a high molecular weight ε-caprolactone homopolymer, or a copolymer derived from a major amount of ε-caprolactone and a minor amount of a comonomer or a blend of such an ε-caprolactone polymer with a lubricating agent (e.g., sterol esters of fatty acids).

Copolymers derived from ε-caprolactone and at least one other monomer such as glycolide, lactide, p-dioxanone and trimethylene carbonate are disclosed in U.S. Patent Nos. 4,605,730, 4,624,256, 4,700,704, 4,788,979, 4,791,929, 4,994,074, 5,076,807, 5,080,665, 5,085,629 and 5,100,433.

U.S. Patent No. 3,896,814 discloses a dry-packaged gut suture which is coated with a treatment agent such as polyoxyethylene glycol. U.S. Patent No. 4,027,676 discloses a gut suture coated with a three-component coating composition. This patent discloses polyalkylene glycol as one ingredient of the three-component coating composition. U.S. Patent No. 4,506,672 discloses a gut suture coated with a cured isocyanate-capped polyester which can be packaged either dry or in alcohol-containing wrappers. U.S. Patent No. 4,649,920 discloses an absorbable surgical suture coated with a high molecular weight poly(alkylene oxide).

The aforementioned U.S. Patent Nos. 3,896,814, 4,027,676, 4,506,672 and 4,649,920 do not disclose a gut suture which is coated with an ε-caprolactone-containing bioabsorbable copolymer.

EP-A-628 587 discloses a bioabsorbable copolymer obtainable by polymerizing a major amount of epsilon-caprolactone and a minor amount of at least one copolymerizable monomer in the presence of polyhydric alcohol as initiator.

US 5 037 429 describes a method for improving the storage stability of a bioabsorbable polymeric braided surgical suture fabricated from a polymer susceptible to hydrolysis which comprises applying a storage stabilizing amount of glycerol to a braided surgical suture formed from said polymer.

<u>SUMMARY</u>

A gut suture is coated with a composition comprising a bioabsorbable copolymer obtained by polymerizing a major amount of ε-caprolactone and a minor amount of at least one other copolymerizable monomer in the presence of a polyhydric alcohol initiator.

The use of a polyhydric alcohol initiator, i.e., an alcohol possessing three or more hydroxyl groups, provides a copolymer having a branched, or "star", configuration. The branched structure of the bioabsorbable copolymer exerts a characteristic influence on its bioabsorption behavior making it useful as a coating material for gut sutures.

The gut suture coated with the bioabsorbable copolymer is packaged in the dry state, i.e., in the absence of tubing fluid, and yet still maintain substantially the same degree of flexibility, pliability and resistance to fray exhibited by a gut suture which is stored in tubing fluid. Thus, a gut suture coated with a coating composition in accordance with this disclosure can be packaged in a manner which is typical for conventional surgical sutures and, when removed from its package, be immediately employed by the surgeon.

In an alternative embodiment, a pre-coating composition is applied to the gut suture prior to being coated with a bioabsorbable copolymer in accordance with this disclosure. A preferred pre-coating composition includes poly(alkylene oxide) such as polyethylene glycol.

BRIEF DESCRIPTION OF THE DRAWING

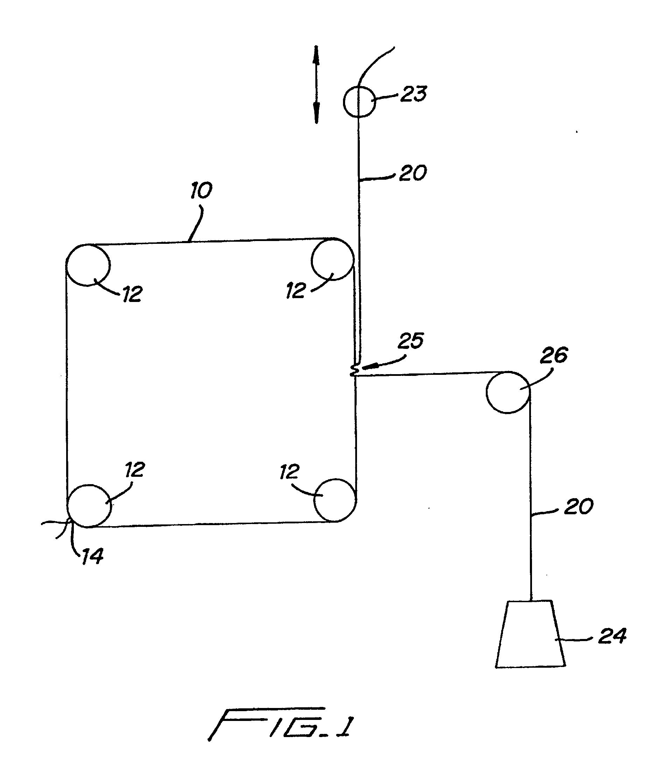

Fig. 1 schematically illustrates a fray testing system for sutures.

DETAILED DESCRIPTION OF PREFERRED EMBODIMENTS

Conventional polymerization techniques that are well known and disclosed in the prior art can be utilized in preparing the bioabsorbable copolymer employed as a coating composition for a gut suture. The bioabsorbable copolymer is obtained by polymerizing a major amount of ε-caprolactone and a minor amount of at least one other copolymerizable monomer or mixture of such monomers in the presence of a polyhydric alcohol initiator. The polymerization of these monomers contemplates all of the various types of monomer addition, i.e., simultaneous, sequential, simultaneous followed by sequential, sequential followed by simultaneous, etc.

Suitable monomers which can be copolymerized with ε-caprolactone include glycolide, lactide, p-dioxanone and trimethylene carbonate.

Suitable polyhydric alcohol initiators include glycerol, trimethylolpropane, 1,2,4-butanetriol, 1,2,6-hexanetriol, triethanolamine, triisopropanolamine, erythritol, threitol, pentaerythritol, ribitol, arabinitol, xylitol, N,N,N',N'-tetrakis (2-hydroxyethyl)ethylenediamine, N,N,N',N'-tetrakis(2-hydroxypropyl)ethylenediamine, dipentaerythritol, allitol, dulcitol, glucitol, altritol, iditol, sorbitol, mannitol, inositol, and the like.

The copolymer can contain from about 70 to about 98, and preferably from about 80 to about 95, weight percent E-caprolactone-derived units, the balance of the copolymer being derived from the other copolymerizable monomer(s). The inherent viscosity of the copolymer generally ranges from about 0.10 to about 0.60, and preferably from about 0.20 to about 0.50, dl/g when measured in chloroform at a concentration of 0.2500 g/dl at 30°C. The polyhydric alcohol initiator is generally employed in small amounts, e.g., from about 0.5 to about 5, and preferably from about 0.1 to about 2, weight percent of the total monomer mixture.

The bioabsorbable copolymer can be applied to a gut suture by any suitable process, e.g., by passing the gut suture through a solution of the copolymer, e.g., in acetone, methylene chloride, etc., past a brush or other coating solution applicator, or past one or more spray nozzles dispensing the gut suture coating solution. The gut suture wetted with the coating solution is subsequently air dried and/or passed through or held in a drying oven for a time and at a temperature sufficient to vaporize and drive off the solvent. Preferably, the coated gut suture is first air dried and then dried in an oven maintained at a temperature of about 50°C. The solution of bioabsorbable copolymer can contain a suitable amount of water or other moisturizing agent which swells the gut suture and imparts a desirable degree of flexibility and pliability to the suture. The bioabsorbable copolymer will entrap the moisture within the suture and/or enhance the retention of the moisture within the suture. If desired, the gut suture coating composition can optionally contain additional components, e.g., dyes, antibiotics, antiseptics, growth factors, anti-inflammatory agents, etc. The amount of coating composition applied to a gut suture will vary depending upon the structure of the suture, e.g., the number of filaments, tightness of braid or twist, the size of the suture and its composition.

In an alternative embodiment herein, a gut suture is pre-coated with a pre-coating composition prior to being coated with the bioabsorbable copolymer disclosed herein. Examples of pre-coating compositions which can be employed herein include compositions containing fatty acids, esters and ethers of fatty acids, polyalcohols, fatty alcohols, glycerine, glycols and derivatives thereof and poly(alkylene oxides). Pre-coating compositions containing poly(alkylene oxides) are preferred. Particularly preferred are poly(alkylene oxides) or derivatives thereof having molecular weights ranging from about 300 to about 5000. Examples of poly(alkylene oxides) which can be employed include polyethylene glycol, polypropylene glycol and polyethylene glycol methyl ether. Such pre-coating compositions can generally be applied to the gut suture at a level of from about 0.1 to about 10 weight percent or more and preferably from about 0.5 to about 5 weight percent, based on the final weight of the coated gut suture. The pre-coating composition can be applied to a gut suture by simply immersing the suture in a solution or suspension containing the pre-coating composition and drying the suture. The solution or suspension can contain water which swells the gut suture and becomes entrapped therein by virtue of the coating of the pre-coating composition. Optionally, the gut suture can be immersed in water and/or moisturizing agent to render the suture flexible and pliable prior to contacting the suture with the solution/suspension containing pre-coating composition.

The bioabsorbable copolymer can be applied to the suture after application of the pre-coating composition. The amount of bioabsorbable copolymer applied to a gut suture which has been pre-coated can range from about 0.2 to as much as about 3 weight percent or more and preferably from about 0.5 to about 2 weight percent. For a gut suture which has not been pre-coated, the bioabsorbable copolymer can be applied at a level of from about 0.5 to about 4 weight percent or more and preferably from about 1 to about 3 weight percent. As a practical matter, it is generally preferred to apply the minimum amount of coating composition consistent with good tie-down performance. This level of coating can be readily determined employing routine experimental procedures.

The following examples should be considered as illustrative and not as limitations of the present description. The examples demonstrate that coating formulations containing bioabsorbable copolymer and pre-coating composition as disclosed herein enhance the properties of gut sutures coated therewith.

Formulation 1

Dry glycolide (300g), $\varepsilon$-caprolactone (2760g), stannous octoate as catalyst (0.3g) and dry mannitol as initiator (39.0g) were mixed under $N_2$ for one hour. The mixture was heated in a reactor at a temperature of 160°C for 24 hours. A solution of the resultant copolymer was prepared by dissolving the copolymer (5g) in toluene (95cc) and stirring the resultant mixture.

Formulation 2

A solution of polyethylene glycol methyl ether 350 (PEGME 350) (number average molecular weight of 350 and viscosity of 4.1 centistokes at 210°F) was prepared by mixing PEGME 350 (50cc) in isopropyl alcohol (50cc) and stirring the resultant mixture.

Formulation 3

A solution of polyethylene glycol methyl ether 350 was prepared by mixing PEGME 350 (as used in Formulation 2) (70cc) in isopropyl alcohol (30cc) and stirring the resultant mixture.

Formulation 4

A solution of polyethylene glycol methyl ether 350 was prepared by mixing 60 cc of PEGME 350 (as used in Formulation 2) with 40 cc of a solution made from 20% water and 80% isopropyl alcohol.

Formulation 5

A solution of polyethylene glycol 600 (PEG 600) (number average molecular weight of 600 and viscosity of 10.5 centistokes at 210°F) was prepared by mixing 60 cc of PEG 600 in 40 cc of a solution made from 20% water and 80% isopropyl alcohol.

EXAMPLE 1

Chrome gut sutures of size 1 are passed through a 10% solution of the copolymer of Formulation 1 in methylene chloride. The sutures are then air dried to remove the solvent, leaving a coating of the copolymer on the suture.

EXAMPLE 2

A chrome size 1 gut suture was dipped in the solution of Formulation 2 for 30 minutes, air dried and thereafter dried in an oven at 50°C for 5 minutes. The suture was then dipped in the solution of Formulation 1 for about 1 minute, air dried for 120 minutes and oven-dried at 50°C for 5 minutes. The resulting suture was then packaged dry and tested for number of cycles to break.

EXAMPLE 3

A chrome size 1 gut suture was dipped in the solution of Formulation 3 for 30 minutes, air dried and thereafter dried in an oven at 50°C for 5 minutes. The suture was then dipped in the solution of Formulation 1 for about 1 minute, air dried for 120 minutes and oven-dried at 50°C for 5 minutes. The resulting suture was then packaged dry and tested for number cycles to break.

EXAMPLE 4

A chrome size 1 gut suture was immersed in the solution of Formulation 4 for 30 minutes at 50°C, air dried for 60 minutes and thereafter immersed in the solution of Formulation 1 for about 1 minute. The suture was then removed, air dried for 120 minutes and oven-dried at 50°C for 5 minutes. The resulting suture was then packaged dry and tested for number of cycles to break.

EXAMPLE 5

A chrome size 1 gut suture was immersed in the solution of Formulation 5 for 30 minutes at 50°C, air dried for 60 minutes and thereafter immersed in the solution of Formulation 1 for about 1 minute. The suture was removed, air dried for 120 minutes and oven-dried at 50°C for 5 minutes. The resulting suture was then packaged dry and tested for number of cycles to break.

COMPARATIVE EXAMPLE 1

A chrome size 1 gut suture was immersed in isopropyl alcohol for 30 minutes at 50°C, air dried for 60 minutes and thereafter immersed in toluene for one minute. The coated suture was removed, air dried for 120 minutes and oven-dried at 50°C for 5 minutes. Thus, this gut suture contains no polymeric coating composition. The suture was packaged dry and tested for number of cycles to break.

Table I below presents the data which resulted from tests conducted on the coated gut sutures of Examples 2-5 and Comparative Example 1. Tensile strength was tested in accordance with the test procedure described in ASTM D-2256. Percent elongation was tested in accordance with the test procedure described in ASTM D-2256. Young's Modulus, which is a measurement of flexibility, is the initial modulus as determined from the slope of the stress-strain curves produced in straight-pull strength tests carried out in accordance with the test procedure described in ASTM D-2256. Young's Modulus is the ratio of applied stress to strain in the elastic region.

Table I

| Young's | | | Tensile | | |
|---|---|---|---|---|---|
| Modulus Example | Number of Cycles to Break | Standard Deviation | Strength (kpsi) | Percent Elongation | (kpsi) |
| 2 | 52.7 | 11.0 | 75 | 21.3 | 178 |
| 3 | 28.4 | 34.0 | 76 | 21 | 89.7 |
| 4 | 17.7 | 13.0 | 83 | 20.5 | 208 |
| 5 | 44.5 | 12.0 | 81 | 21.6 | 207 |
| Comparative Example 1 | 0.3 | 0.2 | 79.2 | 18.9 | 199.7 |

The number of cycles needed to break each of the sutures of Examples 2-5 and Comparative Example 1 was determined using the fray resistance test schematically illustrated in Fig. 1. A static suture 10 is wound around rollers 12 and tied into a knot 14. A dynamic suture 20 is placed into a grip 23 and extended to reach the static suture 10 where it is wrapped twice at point 25 around the static suture 10. The dynamic suture 20 is extended around roller 26 and attached to a weight 24 which supplies tension to the dynamic suture 20. The grip 23 and dynamic suture 20 move up and down to cause the sutures to rub against each other at point 25. One cycle is a complete up and down movement of the grip 23 and dynamic suture 20. Testing conditions included a preload weight which is 15% of the USP limit on average knot pull strength for gut sutures. The travel distance for the grip was 50mm for each cycle at a speed of 500mm/minute. The test is dependent on the number of cycles needed to break a suture due to the fraying which occurs when one strand of suture, under applied load, slides against another static strand. A modified Sintech 1/G MTS system tester is used to conduct the fraying test. The bottom grip is removed from the tester, the load calibrated and gage set to zero. The static suture 10 is tied with sufficient tension around the rollers 12 of the fixture, forming a square. The fixture is adjusted so the point 25 where the static suture 10 and dynamic suture 20 interface is in line with the center line of the upper grip. The preload weight for these examples was 0.550 (15% of USP knot pull, kg.). The test is initiated with cycling observed until one of the sutures breaks to stop the test. If strands should lock themselves in a knot and do not slide against each other it is considered a break. The average number of cycles ($X_{ave.}$) is $X_{ave.} = (X_1 + X_2 + .... + X_n)/n$ wherein $X_n$ is the number of cycles to break each pair of strands and n is the number of pairs. The standard deviation s is calculated as

$$s = \frac{(x_1 - x)^2}{n-1.}$$

It can clearly be seen from the data of Table I that the average number of cycles to break the dry packaged gut sutures coated in accordance with this disclosure (Examples 2-5) were much higher relative to the uncoated dry packaged gut suture of Comparative Example 1. Furthermore, it can be seen from these data that the tensile strength, percent elongation and Young's modulus of each of the dry packaged coated sutures of Examples 2-5 remained comparable to that of the uncoated dry packaged suture of Comparative Example 1.

It will be understood that various modifications may be made to the embodiments disclosed herein. For example, the pre-coating composition can be applied to a gut suture after the bioabsorbable copolymer described herein has been applied to the suture.

The claims which follow identify embodiments of the invention additional to those described in detail above.

## Claims

1. A dry packaged gut suture coated with a coating composition comprising a bioabsorbable copolymer obtained by polymerizing a major amount of ε-caprolactone and a minor amount of at least one other copolymerizable monomer in the presence of polyhydric alcohol as initiator.

2. The gut suture of Claim 1 wherein the other copolymerizable monomer is selected from glycolide, lactide, p-dioxanone and trimethylene carbonate.

3. The gut suture of Claim 1 wherein the polyhydric alcohol initiator is selected from glycerol, trimethylolpropane, 1,2,4-butanetriol, 1,2,6-hexanetriol, triethanolamine, triisopropanolamine, erythritol, threitol, pentaerythritol, ribitol, arabinitol, xylitol, N,N,N',N'-tetrakis(2-hydroxyethyl)ethylenediamine, N,N,N',N'-tetrakis(2-hydroxypropyl)ethylenediamine, dipentaerythritol, allitol, dulcitol, glucitol, altritol, iditol, sorbitol, mannitol and inositol.

4. The gut suture of Claim 1, 2 or 3 wherein the copolymer contains from 70 to 98 weight percent ε-caprolactone-derived units, the balance of the copolymer being derived from the other copolymerizable monomer(s).

5. The gut suture of Claim 4 wherein the copolymer contains from 80 to 95 weight percent ε-caprolactone-derived units, the balance of the copolymer being derived from the other copolymerizable monomer(s).

6. The gut suture of any one of the preceding claims wherein the copolymer possesses an inherent viscosity of from 0.10 to 0.60 dl/g when measured in chloroform at a concentration of 0.2500 g/dl at 30°C.

7. The gut suture of any one of the preceding claims wherein the copolymer possesses an inherent viscosity of from 0.20 to 0.50 dl/g when measured in chloroform at a concentration of 0.2500 g/dl at 30°C.

8. The gut suture of any one of the preceding claims wherein the polyhydric alcohol initiator is employed in an amount of from 0.5 to 5 weight percent of the total monomer mixture.

9. The gut suture of any one of claims 1 to 7 wherein the polyhydric alcohol initiator is employed in an amount of from 0.1 to 2 weight percent of the total monomer mixture.

10. The gut suture of any one of the preceding claims wherein the coating composition is applied to a gut suture at a level of from 0.2 to 4 weight percent of the entire coated suture.

11. The gut suture of Claim 10 wherein the coating composition is applied to a gut suture at a level of from 0.5 to 3 weight percent of the entire coated suture.

12. The gut suture of any one of the preceding claims wherein the suture is precoated with a pre-coating composition comprising fatty acids, esters and ethers of fatty acids, polyalcohols, fatty alcohols, glycerine, glycols and derivatives thereof and poly(alkylene oxides) prior to application of the coating composition.

13. The gut suture of Claim 12 wherein the pre-coating composition comprises a poly(alkylene oxide) selected from polyethylene glycol, polypropylene glycol and polyethylene glycol methyl ether.

14. The gut suture of Claim 13 wherein the poly(alkylene oxide) possesses a molecular weight which ranges from 300 to 5000.

**Patentansprüche**

1. Trocken verpackte Katgut-Naht, beschichtet mit einer Beschichtungszusammensetzung, umfassend ein bioabsorbierbares Copolymer, erhalten durch Polymerisation einer Hauptmenge eines ε-Caprolactons und einer Nebenmenge mindestens eines anderen copolymerisierbaren Monomers in Gegenwart eines mehrwertigen Alkohols als Initiator.

2. Katgut-Naht gemäss Anspruch 1, wobei das andere copolymerisierbare Monomer ausgewählt ist aus Glykolid, Lactid, p-Dioxanon und Trimethylencarbonat.

3. Katgut-Naht gemäss Anspruch 1, wobei der mehrwertige Alkoholinitiator ausgewählt ist aus Glycerin, Trimethylolpropan, 1,2,4-Butantriol, 1,2,6-Hexantriol, Triethanolamin, Triisopropanolamin, Erythritol, Threitol, Pentaerythritol, Ribitol, Arabinitol, Xylitol, N,N,N',N'-Tetrakis(2-hydroxyethyl)ethylendiamin, N,N,N',N'-Tetrakis(2-hydroxypropyl)ethylendiamin, Dipentaerythritol, Allitol, Dulcitol, Glucitol, Altritol, Iditol, Sorbit, Mannit und Inosit.

4. Katgut-Naht gemäss Anspruch 1, 2 oder 3, wobei das Copolymer 70 bis 98 Gew.% von ε-Caprolacton abgeleitete Einheiten enthält, wobei die Balance des Copolymers von den anderen copolymerisierbaren Monomeren (dem Monmer) abgeleitet ist.

5. Katgut-Naht gemäss Anspruch 4, wobei das Copolymer 80 bis 95 Gew.% von ε-Caprolacton abgeleitete Einheiten enthält, wobei die Balance des Copolymers abgeleitet ist von dem anderen copolymerisierbaren Monomer (den Monomeren).

6. Katgut-Naht gemäss einem der vorstehenden Ansprüche, wobei das Copolymer eine innere Viskosität von 0,10 bis 0,60 dl/g besitzt, wenn diese in Chloroform bei einer Konzentration von 0,2500 g/dl bei 30°C gemessen wird.

7. Katgut-Naht gemäss einem der vorstehenden Ansprüche, wobei das Copolymer eine innere Viskosität von 0,20 bis 0,50 dl/g besitzt, wenn diese in Chloroform bei einer Konzentration von 0,2500 g/dl bei 30°C gemessen wird.

8. Katgut-Naht gemäss einem der vorstehenden Ansprüche, wobei der mehrwertige Alkoholinitiator in einer Menge von 0,5 bis 5 Gew.% der Gesamtmonomermischung verwendet wird.

9. Katgut-Naht gemäss einem der Ansprüche 1 bis 7, wobei der mehrwertige Alkoholinitiator in einer Menge von 0,1 bis 2 Gew.% der Gesamtmonomermischung angewandt wird.

10. Katgut-Naht gemäss einem der vorstehenden Ansprüche, wobei die Beschichtungszusammensetzung einer Katgut-Naht mit einem Anteil von 0,2 bis 4 Gew.% der gesamten beschichteten Naht aufgebracht wird.

11. Katgut-Naht gemäss Anspruch 10, wobei die Beschichtungszusammensetzung einer Katgut-Naht in einem Anteil von 0,5 bis 3 Gew.% der gesamten beschichteten Naht aufgebracht wird.

12. Katgut-Naht gemäss einem der vorstehenden Ansprüche, wobei die Naht mit einer Vorbeschichtungszusammensetzung, umfassend Fettsäuren, Ester und Ether von Fettsäuren, Polyalkohole, Fettalkohole, Glycerin, Glykole und Derivate davon und Poly(alkylenoxide), vor dem Aufbringen der Beschichtungszusammensetzung vorbeschichtet wird.

**13.** Katgut-Naht gemäss Anspruch 12, wobei die Vorbeschichtungszusammensetzung ein Poly(alkylenoxid) umfasst, ausgewählt aus Polyethylenglykol, Polypropylenglykol und Polyethylenglykolmethylether.

**14.** Katgut-Naht gemäss Anspruch 13, wobei das Poly(alkylenoxid) ein Molekulargewicht besitzt, das im Bereich von 300 bis 5.000 liegt.

**Revendications**

**1.** Fil pour sutures en boyau emballé à sec avec une composition de revêtement comprenant un copolymère bioabsorbable obtenu par polymérisation d'une quantité majeure d'ε-caprolactone et d'une quantité mineure d'au moins un autre monomère copolymérisable en présence d'alcool polyhydrique comme initiateur.

**2.** Fil pour sutures en boyau de la revendication 1, où l'autre monomère copolymérisable est sélectionné parmi glycolide, lactide, p-dioxanone et triméthylène carbonate.

**3.** Fil pour sutures en boyau de la revendication 1, où l'alcool polyhydrique initiateur est sélectionné parmi glycérol, triméthylolpropane, 1,2,4-butanetriol, 1,2,6-hexanetriol, triéthanolamine, triisopropanolamine, érythritol, thréitol, pentaérythritol, ribitol, arabinitol, xylitol, N,N,N', N'-tétrakis(2-hydroxyéthyl) éthylènediamine, N,N,N',N'-tétrakis (2-hydroxypropyl)éthylènediamine, dipentaérythritol, allitol, dulcitol, glucitol, altritol, iditol, sorbitol, mannitol et inositol.

**4.** Fil pour sutures en boyau de la revendication 1, 2 ou 3, où le copolymère contient de 70 à 98 pour cent en poids d'unités dérivées d'ε-caprolactone, le reste du copolymère étant dérivé du ou des autres monomères copolymérisables.

**5.** Fil pour sutures en boyau de la revendication 4, où le copolymère contient de 80 à 95% en poids d'unités dérivées d'ε-caprolactone, le reste du copolymère étant dérivé du ou des autres monomères copolymérisables.

**6.** Fil pour sutures en boyau de l'une quelconque des revendications précédentes, où le copolymère possède une viscosité inhérente de 0,10 à 0,60 dl/g en mesurant dans le chloroforme à une concentration de 0,2500 g/dl à 30°C.

**7.** Fil pour sutures en boyau de l'une quelconque des revendications précédentes, où le copolymère possède une viscosité inhérente de 0,20 à 0,50 dl/g en mesurant dans le chloroforme à une concentration de 2500 g/dl à 30°C.

**8.** Fil pour sutures en boyau de l'une quelconque des revendications précédentes, où l'alcool polyhydrique initiateur est employé en une quantité de 0,5 à 5% en poids du mélange total de monomères.

**9.** Fil pour sutures en boyau de l'une quelconque des revendications 1 à 7, où l'alcool polyhydrique initiateur est employé en une quantité de 0,1 à 2 pour cent en poids du mélange total de monomères.

**10.** Fil pour sutures en boyau de l'une quelconque des revendications précédentes, où la composition de revêtement est appliquée à un fil pour sutures en boyau à un niveau de 0,2 à 4 pour cent en poids de toute la suture revêtue.

**11.** Fil pour sutures en boyau de la revendication 10, où la composition de revêtement est appliquée à un fil pour sutures en boyau à niveau de 0,5 à 3 pour cent en poids de toute la suture revêtue.

**12.** Fil pour sutures en boyau de l'une quelconque des revendications précédentes, où la suture est pré-enduite d'une composition de pré-induction comprenant des acides gras, des esters et éthers d'acides gras, des polyalcools, des alcools gras, de la glycérine, du glycol et leurs dérivés et des poly(oxydes d'alkylène) avant application de la composition de revêtement.

**13.** Fil pour sutures en boyau de la revendication 12, où la composition pré-revêtement comprend un poly(oxyde d'alkylène) sélectionné parmi polyéthylène glycol, polypropylène glycol et polyéthylène glycol méthyl éther.

**14.** Fil pour sutures en boyau de la revendication 13, où le poly(oxyde d'alkylène) possède un poids moléculaire qui va de 300 à 5000.

FIG.1